# EUROPEAN PATENT APPLICATION

(11) **EP 2 815 743 A1**
(43) Date of publication of application: **24.12.2014**
(21) Application number: 14173223.0
(22) Date of filing: 20.06.2014
(51) Int. Cl.: A61K 9/46, A61K 31/546

(54) **Ceftibuten formulations**

(30) Priority: 21.06.2013 TR 201307512
(71) Applicant: Sanovel Ilac Sanayi ve Ticaret A.S., 34460 Istanbul (TR)
(72) Inventor: Türkyilmaz, Ali, 34460 Istanbul (TR); Karabulut, Tutku Ceren, 34460 Istanbul (TR); Yaman, Bircan, 34460 Istanbul (TR); Demir, Bülent, 34460 Istanbul (TR)
(74) Representative: Sevinç, Erkan

(57) **Abstract**

The present invention relates to a pharmaceutical formulation comprising ceftibuten or a pharmaceutically acceptable form thereof and pullulan. The present invention further relates to a production method of said pharmaceutical formulation and to the use thereof in the prevention or treatment of bacterial infections.

## Description

### Field of Invention

The present invention relates to a pharmaceutical formulation comprising ceftibuten or a pharmaceutically acceptable form thereof and pullulan. The present invention further relates to a production method for said pharmaceutical formulation and to the use thereof in the prevention or treatment of bacterial infections.

### Background of Invention

Ceftibuten, with the chemical name (+)-(6*R*,7*R*)-7-((*Z*)-2-(2-amino-4-thiazolyl)-4-carboxy-crotonamido)-8-oxo-5-thia-1-azabicyclo[4.2.0]-oct-2-ene-2-carboxylic acid, is a third-generation cephalosporin antibiotic. On the other hand, ceftibuten is a β-lactam antibiotic with bactericidal effect used in the prevention and treatment of bacterial infections. The chemical structure of ceftibuten is illustrated in Formula I given below.

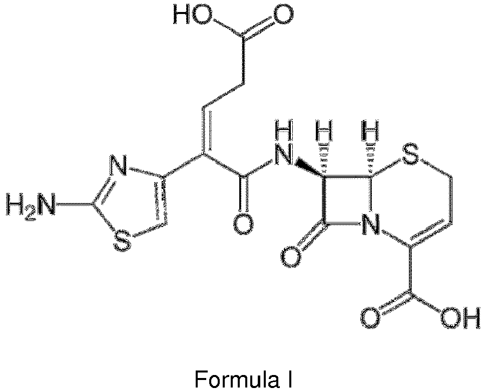

Ceftibuten binds to specific penicillin-binding proteins which are present on the inner surface of the bacterial cell wall and provides a bactericidal effect by inhibiting the third and final stage of the bacterial cell wall. It is known to be quite effective particularly against Gram-negative bacteria, but is also used in the prevention and treatment of some diseases caused by some Gram-positive bacteria. The bacteria on which ceftibuten is effective may be exemplified with *Haemophilus influenzae, Moraxella catarrhalis, Escherichia coli (K. pneumoniae, K. oxytoca), Proteus vulgaris, P. mirabils, P. providence, Salmonella* sp., *Shigella* sp., *Enterobacter* sp. and *Streptococcus sp..*

The ceftibuten molecule was first disclosed in the patent US 4,634,697. That patent further described the antibacterial effect of the ceftibuten molecule.

US 4,933,443 discloses dihydrate and trihydrate crystals of ceftibuten and a method for producing the same; US 5,017,380 discloses hard gelatin capsules comprising hydrate crystals of ceftibuten; and US 4,812,561 discloses a crystalline hydrate of ceftibuten.

The patent application EP1905432 discloses a stable nanoparticulate composition comprising cephalosporin particles having a diameter size less than 2000 nm and at least one surface stabilizer adsorbed on the surface of the particles, said surface stabilizer being free of intermolecular cross-linkages.

The patent application WO2011093825A2 discloses a process for the preparation of an effervescent pharmaceutical composition comprising at least one cephalosporin antibiotic. According to that application, the composition is prepared using a wet granulation process, involving the use of a granulation solution comprising an organic solvent and water in an amount of 5-25%.

The patent EP0890359B1 discloses a tablet formulation comprising 60 wt.% to 85 wt.% beta-lactam antibiotic, 1 wt.% to 10 wt.% hydroxypropyl cellulose and/or crosslinked polyvinylpyrrolidone, and 0.5 wt.% to 2 wt.% polyvinylpyrrolidone, hydroxypropyl cellulose or hydroxypropyl methylcellulose.

The patent application EP1458398A1 discloses an antibiotic formulation comprising at least one beta-lactam antibiotic and N-methyl-2-pyrrolidone, wherein said antibiotic is dissolved in N-methyl-2-pyrrolidone.

The patent application EP2566449A1 discloses a pharmaceutical composition comprising at least one non-cellulosic binder having a mean particle size between 60 µm and 150 µm, wherein the ratio of ceftibuten to this binder varies between 7:1 and 4:1.

Being effective against bacterial infections, ceftibuten is weakly soluble in water and therefore it is quite difficult to formulate it in an effervescent form. Based on this feature of ceftibuten, it is typically produced in the form of tablets, capsules, and suspensions. However, some advantages of effervescent formulations over other dosage forms gave rise to a need for producing the effervescent formulations of ceftibuten.

One of the most important advantages of effervescent formulations is that the effect of the active agent has a rapid onset. Since effervescent tablets are dissolved in water prior to administration, the active agent is absorbed easily so that the active agent shows its effect in a short time period. Having said that, the pH value of a solution obtained by dissolving the effervescent formulation in water is set to a suitable level in terms of the absorption of the active agent by the stomach using buffering agents therein, and this contributes to an easy absorption of the active agent delivered to the stomach. Administering effervescent formulations in the form of a buffered solution reduces the local contact of the active agent with the upper gastrointestinal tract and therefore this results in less irritation and more tolerability.

An effervescent formulation is taken easier than a tablet or capsule as it is administrated in an aqueous form. For this reason, administering the active agent in an effervescent form to those patients who don't like to take tablets or capsules or who have difficulties in swallowing tablets or capsules increases the compliance of the patients to the treatment and ensures to maintain the treatment in an efficient manner.

Additionally, since effervescent formulations are normally in a solid form and dissolved in a liquid, particularly water, just before administration, these formulations are carried more easily than liquid drugs. Furthermore, sweeteners and/or taste masking agents provided in effervescent formulations facilitate the administration of even those active agents which have a bitter or unpleasant taste. In addition, effervescent formulations are more stable and have such pharmacokinetic profiles which are more expectable and reproducible as compared to liquid formulations.

In other respects, effervescent formulations make it possible to administer a higher dose of drug at a time. In general terms, an effervescent tablet can comprise an amount of active agent which almost corresponds to an active agent amount comprised by around three conventional tablets. Therefore, administering the active agent in an effervescent form provides ease of use to a patient when he/she is required to take a high drug dose at a time or to take more than one drug a day.

Researchers have proven that effervescent formulations increase the absorption of many active agents when compared to conventional formulations. In this context, the carbon dioxide gas released during an effervescent reaction changes the intercellular distance and stimulates the permeability of the active agent. It is thus considered that carbon dioxide expands the intercellular gaps so that the absorption of hydrophobic or hydrophilic active agents is increased. Accordingly, effervescent formulations are formulated aiming to improve the therapeutic profile of the respective active agents comprised.

Although effervescent formulations have the aforesaid advantages, it is quite difficult to prepare an effervescent formulation of ceftibuten because of its low water-solubility. The low solubility and dissolution rate of ceftibuten cause the effervescent formulations of ceftibuten to have a low bioavailability. This is because a drug has to be completely dissolved in a liquid (e.g. water) and absorbed following administration to provide a high bioavailability. Accordingly, an incomplete dissolution of an effervescent drug lowers down the absorption and therefore the bioavailability of the drug.

In order to maintain good quality and shelf life for effervescent formulations comprising ceftibuten, it is also important to maintain the stability thereof beside providing a high bioavailability. Effervescent formulations of ceftibuten can undergo chemical and structural changes under environmental and physical influences and their stability may be deteriorated. This, in turn, causes the formulation to show chemical and physical changes, to worsen in terms of quality, as well as reduces the shelf life thereof and decreases the therapeutic activity of the active agent. Therefore, both a high bioavailability and a long-term stability have to be targeted in the development of effervescent formulations of ceftibuten.

In addition, ceftibuten is a cephalosporin antibiotic with a bitter taste. Formulating the effervescent formulations of ceftibuten in a manner that would provide an easy and regular administration in terms of patients will naturally increase the compliance of the patients to the treatment and the efficiency of the treatment itself.

Under the light of the foregoing, there is obviously a need for an effervescent formulation of ceftibuten, having a high solubility, dissolution rate, and therefore a high bioavailability, a long-term stability, and a good taste.

### Brief Description of Invention

The present invention relates to an effervescent formulation of ceftibuten, eliminating all aforesaid problems and bringing additional advantages to the relevant prior art.

The main object of the present invention is to provide an effervescent formulation of ceftibuten, which has a high solubility and dissolution rate, and therefore an improved absorption and bioavailability.

Another object of the present invention is to provide an effervescent formulation of ceftibuten, which is resistant against environmental and physical influences, has a long-term stability and therefore a long shelf life.

A further object of the present invention is to provide an effervescent formulation of ceftibuten, which has an improved taste for improving the patient compliance and increasing the treatment efficiency, beside those features specified above.

Another object of the present invention is to provide an easily-applicable method for preparing an effervescent formulation of ceftibuten.

In order to achieve all objects stated above and described in the following detailed description, an effervescent formulation is developed which comprises ceftibuten or a pharmaceutically acceptable form of ceftibuten.

According to an embodiment of the present invention, the effervescent formulation according to the present invention comprises ceftibuten or a pharmaceutically acceptable form of ceftibuten and pullulan.

According to another preferred embodiment of the present invention, the amount of pullulan in the effervescent formulation is 0.05% to 25% by weight, preferably 0.1% to 15% by weight of the total weight of the formulation.

According to another preferred embodiment of the present invention, the ratio by weight of ceftibuten to pullulan in the effervescent formulation is between 1:10 and 100:1, preferably between 1:1 and 50:1.

According to another preferred embodiment of the present invention, the amount of ceftibuten in the effervescent formulation is 10% to 90% by weight, preferably 15% to 75% by weight of the total weight of the formulation.

According to another preferred embodiment, the effervescent formulation according to the present invention further comprises at least one pharmaceutically acceptable excipient selected from a group comprising binders, sweeteners, glidants, effervescent pairs, surface active agents, disintegrants, flavoring agents, and viscosity agents, besides ceftibuten and pullulan.

According to another preferred embodiment, the effervescent formulation according to the present invention further comprises sucralose as a sweetener, besides ceftibuten and pullulan.

According to another preferred embodiment of the present invention, the amount of sucralose in the effervescent formulation is 0.01% to 15% by weight, preferably 0.05% to 10% by weight of the total weight of the formulation.

According to another preferred embodiment of the present invention, a method is provided for preparing an effervescent formulation of ceftibuten, comprising the steps of
a. weighing defined amounts of ceftibuten, sodium bicarbonate, citric acid, pullulan, sucralose, PEG 8000, orange flavor, lemon flavor, xanthan gum, and sodium lauryl sulfate, introducing these ingredients into a mixer and stirring therein (first mixture),
b. adding a previously weighed amount of sodium stearyl fumarate by sieving into the first mixture and taking the resulting mixture into a mixer and stirring therein (second mixture),
c. subjecting the resulting uniform mixture obtained in the final stirring step to a filling and packaging process.

### Detailed Description of Invention

The absorption and bioavailability of a pharmaceutical effervescent formulation is closely associated to the solubility and dissolution rate of the active agent. Accordingly, providing an increase in the solubility and dissolution rate of an active agent in an effervescent formulation will increase the amount of the active agent to be adsorbed and therefore improve the bioavailability of the formulation. This, in turn, shall increase the therapeutic efficiency of the active agent in the effervescent formulation.

Thus, the present invention provides a novel effervescent formulation of ceftibuten having a surprisingly-improved solubility and dissolution rate for use in the treatment or alleviation of the symptoms of bacterial diseases.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed.

Pullulan is a homopolysaccharide used for various purposes in pharmaceutical formulations and is generally produced extracellularly by *Aureobasidium pullulans,* a yeast-like fungus. Considering the chemical structure, pullulan is a glucan comprising maltotriose units which are connected by α-1,6 glicosidic bonds. It was surprisingly found that using pullulan as an excipient in the effervescent formulation of ceftibuten according to the present invention increases the solubility and the dissolution rate and accordingly the bioavailability of the active agent. Based on its superior physical and chemical properties, pullulan results in a homogeneous solution by increasing the aqueous solubility and dissolution rate of ceftibuten, which is actually weakly soluble in water. Thus, the absorption and therefore the bioavailability of a formulation administrated in the form of a homogeneous solution are increased. Hence, pullulan also contributes to improving the therapeutic efficiency of ceftibuten in the effervescent formulation.

Accordingly, the present invention provides an effervescent formulation of ceftibuten comprising pullulan for use in the treatment, prevention, or in the alleviation of the symptoms of bacterial infections.

The effervescent formulation according to the present invention comprises 0.05% to 25% by weight of pullulan, preferably 0.1 % to 15% by weight of pullulan, and particularly preferably 0.5% to 10% by weight of pullulan based on the total weight of the formulation.

In the effervescent formulation according to the present invention, the ratio by weight of ceftibuten to pullulan is between 1:10 and 100:1, preferably between 1:1 and 50:1, and more preferably between 1:1 and 25:1. Keeping the ratio by weight of ceftibuten to pullulan in the effervescent formulation in the limits stated above surprisingly resulted in improving the stability of the formulation besides bioavailability and contributed to increasing the shelf life of the effervescent formulation. By virtue of the synergistic effect achieved with the superior binding property and low oxygen permeability of pullulan, both the bioavailability and the stability of the effervescent formulation are improved and the therapeutic efficiency of the active agent is prolonged.

The effervescent formulation according to the present invention comprises 10% to 90% by weight of ceftibuten, preferably 15% to 75% by weight of ceftibuten, and more preferably 22% to 50% by weight of ceftibuten based on the total weight of the formulation.

The effervescent formulation according to the present invention comprises ceftibuten or a pharmaceutically acceptable form of ceftibuten as an active agent. In other words, the active agent ceftibuten may be present in the effervescent formulation in an amorphous form, in a crystal form, in a free base form, in a hydrate form, or in the form of a mixture thereof, but it is preferably present in a dihydrate form, in a trihydrate form, or in a mixture of these forms.

The term "a pharmaceutically acceptable form of ceftibuten" as used in the present disclosure describes those forms of ceftibuten which are therapeutically useful.

The particle size of the active agent in the effervescent formulation is one of the most important factors effecting the solubility and the dissolution rate of the active agent. Increasing the surface area of the active agent improves the solubility thereof. Improving the solubility of the active agent, in turn, directly influences the bioavailability of the formulation and enhances the therapeutic efficiency of the active agent. Setting the median particle size of ceftibuten in the effervescent formulation according to the present invention above 2 µm, preferably between 3 µm and 50 µm, more preferably between 4 µm and 25 µm helps to increase the solubility and therefore the absorption of ceftibuten. This, in turn, allows to further increase the bioavailability of the formulation.

The median particle size of ceftibuten in the effervescent formulation according to the present invention refers to d_{0.5} value obtained by a dry dispersion method carried out on a Malvern Mastersizer 2000 Particle Size Analyzer using air as a dispensing agent. The d_{0.5} value according to the present invention is also known as a median particle size by volume and indicates the maximum particle diameter of 50% of particles by volume.

Having said that, other particle size values of ceftibuten (i.e. d_{0.1} and d_{0.9}) in the effervescent formulation according to the present invention are similarly obtained by a dry dispersion method carried out on a Malvern Mastersizer 2000 Particle Size Analyzer using air as a dispensing agent, with the d_{0.1} value being between 0.1 µm and 15 µm, preferably between 0.2 µm and 10 µm, more preferably between 0.5 µm and 5 µm; and the d_{0.9} value being between 30 µm and 180 µm, preferably between 45 µm and 120 µm, and more preferably between 50 µm and 90 µm. Within the scope of the present invention, the value d_{0.1} represents the maximum particle diameter of 10% of particles by volume, and the value d_{0.9} represents the maximum particle diameter of 90% of particles by volume.

The effervescent formulation according to the present invention further comprises at least one pharmaceutically acceptable excipient selected from a group comprising binders, sweeteners, glidants, effervescent pairs, surface active agents, disintegrants, flavoring agents, and viscosity agents.

Suitable lubricants comprise, but are not limited to magnesium stearate, calcium stearate, aluminum stearate, polyethylene glycol, PEG 6000, PEG 8000, talk, sodium stearyl fumarate, polyvinyl alcohol, sodium benzoate, potassium benzoate, silicon, or the mixtures thereof.

Suitable effervescent pairs comprise, but are not limited to combinations of a base source selected from a group comprising sodium carbonate, sodium bicarbonate, potassium carbonate, potassium bicarbonate, sodium sesquicarbonate, sodium glycine carbonate, L-lysine carbonate, calcium carbonate, and an acid source selected from a group comprising citric acid, tartaric acid, ascorbic acid, fumaric acid, malic acid, adipic acid, succinic acid, acetyl salicylic acid, nicotinic acid.

Suitable viscosity agents comprise, but are not limited to glycerin, gelatin, modified starches, xanthan gum, dextran, synthetic or semisynthetic gums, cellulose and the derivatives thereof, chitosan, carbomer, or the mixtures thereof.

Suitable surface active agents comprise, but are not limited to polysorbate (e.g. polysorbate 60), glyceryl monostearate, polyethylene glycol succinate, oleic acid, diethanolamine, sodium lauryl sulfate, propylene glycol, or the mixtures thereof.

Suitable disintegrants comprise, but are not limited to starch, microcrystalline cellulose, crospovidone, silicon dioxide, croscarmellose sodium, sodium starch glycolate, hydrogenated castor oil, carboxymethylcellulose sodium, carboxymethylcellulose calcium, povidone, methyl cellulose, or the mixtures thereof.

Suitable sweeteners comprise, but are not limited to sucralose, ammonium glycyrrhizinate, acesulfame-K, aspartame, saccharine, or sodium salts or calcium salts of saccharine, sodium cyclamate, sucrose, fructose, glucose, sorbitol, or the mixtures thereof, preferred sweetener is sucralose. Sucralose in the effervescent formulation has surprisingly contributed to suppressing the bitter taste of ceftibuten and improving the taste of the formulation without deteriorating the bioavailability and stability of the formulation. Thus, sucralose present in the effervescent formulation improves the taste of the formulation and increase the patient compliance and maintains the progress of the treatment. The effervescent formulation of ceftibuten according to the present invention comprises 0.01 % to 15% by weight of sucralose, preferably 0.05% to 10% by weight of sucralose, more preferably 0.1% to 5% by weight of sucralose, and most preferably 0.1 % to 2% by weight of sucralose based on the total weight of the formulation.

Suitable flavoring agents comprise, but are not limited to fruit flavors like those of orange, cherry, strawberry, banana, sour cherry, lemon, etc., and cardamom, anis, mint, menthol, eucalyptus, vanillin, and ethyl vanillin, or the mixtures thereof.

Besides pullulan, the effervescent formulation according to the present invention can further comprise a binder selected from a group comprising povidone (polyvinylpyrrolidone or polyvidone), dextrose, sorbitol, xylitol, lactose, cellulose, gelatin, hydroxypropyl cellulose, hydroxypropyl methylcellulose, methyl cellulose, starch, pregelatinized starch, arabic gum, or the mixtures thereof.

On the other hand, the present invention is used in the treatment, prevention, or in the alleviation of the symptoms of bacterial infectious diseases in the children and in the adults. The present invention is more specifically used in the treatment, prevention, or in the alleviation of the symptoms of upper respiratory tract infections such as sinusitis, pharyngitis, scarlatina, tonsillitis, otitis media; lower respiratory tract infections such as acute bronchitis, chronic bronchitis, acute exacerbation of chronic bronchitis, acute pneumonia; urinary tract infections such as acute and chronic pyelitis, cystopyelitis, cystitis, urethritis in the children and in the adults.

An effervescent formulation according to the present invention preferably comprises the following ingredients:
a. 10 to 90% by weight of ceftibuten,
b. 15 to 50% by weight of sodium bicarbonate,
c. 10 to 45% by weight of citric acid,
d. 0.05 to 25% by weight of pullulan,
e. 0.01 to 15% by weight of sucralose,
f. 0.5 to 20% by weight of PEG 8000,
g. 0.05 to 15% by weight of orange flavor,
h. 0.05 to 15% by weight of lemon flavor,
i. 0.001 to 5% by weight of sodium stearyl fumarate,
j. 0.05 to 15% by weight of xanthan gum,
k. 0.005 to 5% by weight of sodium lauryl sulfate.

An effervescent formulation according to the present invention preferably comprises the following ingredients:
a. 22 to 50% by weight of ceftibuten,
b. 15 to 50% by weight of sodium bicarbonate,
c. 10 to 45% by weight of citric acid,
d. 0.5 to 10% by weight of pullulan,
e. 0.1 to 2% by weight of sucralose,
f. 0.5 to 20% by weight of PEG 8000,
g. 0.05 to 15% by weight of orange flavor,
h. 0.05 to 15% by weight of lemon flavor,
i. 0.001 to 5% by weight of sodium stearyl fumarate,
j. 0.05 to 15% by weight of xanthan gum,
k. 0.005 to 5% by weight of sodium lauryl sulfate.

The term "effervescent formulations" according to the present invention comprises effervescent tablets, sachets, and effervescent granules. In other words, the effervescent formulations of ceftibuten may in the form of effervescent tablets, sachets, or effervescent granules.

A method for preparing the effervescent formulation in order to achieve another object of the present invention preferably comprises the following steps:
a. weighing defined amounts of ceftibuten, sodium bicarbonate, citric acid, pullulan, sucralose, PEG 8000, orange flavor, lemon flavor, xanthan gum, and sodium lauryl sulfate, introducing these ingredients into a mixer and stirring therein (first mixture),
b. adding a previously weighed amount of sodium stearyl fumarate by sieving into the first mixture and taking the resulting mixture into a mixer and stirring therein (second mixture),
c. subjecting the resulting uniform mixture obtained in the final stirring step to a filling and packaging process.

A method for producing the effervescent formulation according to the present invention preferably comprises the following steps:
a. weighing defined amounts of ceftibuten dihydrate or trihydrate, sodium bicarbonate, citric acid (preferably nonaqueous), pullulan, sucralose, PEG 8000, orange flavor, lemon flavor, xanthan gum and sodium lauryl sulfate, introducing these ingredients into a mixer and stirring therein for 20 minutes (first mixture),
b. adding a previously weighed amount of sodium stearyl fumarate by sieving through a 630 µm sieve into the first mixture and taking the resulting mixture into a mixer and stirring therein for 5 minutes (second mixture),
c. the resulting uniform mixture obtained in the final stirring step is compressed such that each effervescent tablet has preferably a hardness of between 50 N and 250 N, and
d. filling the compressed tablets into blisters and carrying out a packaging step.

If the effervescent formulation according to the present invention is provided in the form of an effervescent tablet, it should have a strength to resistant against the coating, packaging, and transportation steps. For this reason, no friability should occur on the edges or surfaces of the tablet. The strength of the tablet depends on the tablet compression force during production. When the effervescent formulation according to the present invention is compressed under a force of 50 to 250 N, any problems related to the strength of the tablet can be prevented.

### Experimental Data

The dissolution test is performed to demonstrate the effect of the pullulan in dissolution of ceftibuten in comparison with PVP.

An effervescent tablet comprising ceftibuten (400 mg) is dissolved in the water of 150 mL and the sample is taken at 1., 3., 5., 10. and 15. min of dissolution. And the amount of the ceftibuten is determined by HPLC method:
**Column:** YMC Hydrosphere C18 (250mmx4.6mm) 5um
**Flow rate:** 1.0 mL/min
**Injection volume:** 10 µl
**Column temperature:** 30ºC
**Analysis time:** 30 min

The test is repeated for 6 effervescent tablets comprising ceftibuten (400 mg) and pullulan and for another 6 effervescent tablets comprising ceftibuten (400 mg) and PVP (polyvinylpyrrolidone).

**Table 1: The dissolution test results of pullulan**

| ***Time (minute)*** | ***Tablet-1*** | ***Tablet-2*** | ***Tablet-3*** | ***Tablet-4*** | ***Tablet-5*** | ***Tablet-6*** | ***Average*** |
|---|---|---|---|---|---|---|---|
| 0 | 0.00 | 0.00 | 0.00 | 0.00 | 0.00 | 0.00 | 0.00 |
| 1 | 69.51 | 69.57 | 70.02 | 69.77 | 69.82 | 70.19 | 69.81 |
| 3 | 87.81 | 88.15 | 88.17 | 87.98 | 88.21 | 88.10 | 88.07 |
| 5 | 97.68 | 97.58 | 97.14 | 97.28 | 96.90 | 97.15 | 97.29 |
| 10 | 100.63 | 100.59 | 100.53 | 100.42 | 100.44 | 100.65 | 100.54 |
| 15 | 100.44 | 101.07 | 100.87 | 100.78 | 100.90 | 101.38 | 100.91 |

**Table 2: The dissolution test results of PVP**

| ***Time (minute)*** | ***Tablet-1*** | ***Tablet-2*** | ***Tablet-3*** | ***Tablet-4*** | ***Tablet-5*** | ***Tablet-6*** | ***Average*** |
|---|---|---|---|---|---|---|---|
| 0 | 0.00 | 0.00 | 0.00 | 0.00 | 0.00 | 0.00 | 0.00 |
| 1 | 48.91 | 48.82 | 48.79 | 48.59 | 48.58 | 48.43 | 48.69 |
| 3 | 83.30 | 83.80 | 83.96 | 83.78 | 83.86 | 83.98 | 83.78 |
| 5 | 95.25 | 95.15 | 94.90 | 94.86 | 94.40 | 94.39 | 94.82 |
| 10 | 97.59 | 97.51 | 97.36 | 97.27 | 97.19 | 96.82 | 97.29 |
| 15 | 100.96 | 100.94 | 100.34 | 100.54 | 101.08 | 100.67 | 100.75 |

According to Table-1 and Table-2, it is shown that the effervescent tablets comprising ceftibuten and pullulan is dissolved more rapid than the dissolution profile of the effervescent tablets comprising ceftibuten and PVP in the water. Consequently, it is demonstrated that pullulan improves the dissolution of ceftibuten in comparison with PVP.

The present invention is explained in detail in the following examples. These examples are not limiting the scope of the present invention and are to be considered under the light of the foregoing detailed disclosure.

### Examples

### Example 1

Defined amounts of ceftibuten dihydrate with a median particle size (d_{0.5}) of 4 µm to 25 µm as measured using a Malvern Mastersizer 2000 Particle Size Analyzer, sodium bicarbonate, citric acid (preferably nonaqueous), pullulan, sucralose, PEG 8000, orange flavor, lemon flavor, xanthan gum and sodium lauryl sulfate are weighed, these ingredients are introduced into a mixer and stirred therein for 20 minutes (first mixture). A previously weighed amount of sodium stearyl fumarate is added by sieving through a 630 µm sieve into the first mixture and the resulting mixture is taken into a mixer and stirred therein for 5 minutes (second mixture), then the resulting uniform mixture obtained in the final stirring step is compressed such that each effervescent tablet has a hardness of between 50 N and 250 N, and the compressed tablets are filled into blisters and packaged accordingly.

The production method described above can be adapted to all examples given below.

| ***Content of Formulation*** | ***Amount (%)*** |
|---|---|
| Ceftibuten dihydrate | 10% to 90% |
| Sodium bicarbonate | 15% to 50% |
| Citric acid | 10% to 45% |
| Pullulan | 0.05% to 25% |
| Sucralose | 0.05% to 10% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 2

| ***Content of Formulation*** | ***% Range*** |
|---|---|
| Ceftibuten dihydrate | 15% to 75% |
| Sodium bicarbonate | 18% to 45% |
| Citric acid | 12% to 40% |
| Pullulan | 0.1% to 15% |
| Sucralose | 0.1% to 5% |
| PEG 8000 | 1 % to 18% |
| Orange flavor | 0.1 % to 10% |
| Lemon flavor | 0.1 % to 10% |
| Sodium stearyl fumarate | 0.005% to 4% |
| Xanthan gum | 0.1 % to 10% |
| Sodium lauryl sulfate | 0.01% to 4% |

### Example 3

| ***Content of Formulation*** | ***% Range*** |
|---|---|
| Ceftibuten dihydrate | 22% to 50% |
| Sodium bicarbonate | 20% to 40% |
| Citric acid | 15% to 35% |
| Pullulan | 0.5% to 10% |
| Sucralose | 0.1% to 2% |
| PEG 8000 | 2% to 15% |
| Orange flavor | 0.1% to 5% |
| Lemon flavor | 0.1% to 5% |
| Sodium stearyl fumarate | 0.005% to 3% |
| Xanthan gum | 0.1% to 5% |
| Sodium lauryl sulfate | 0.01% to 3% |

### Example 4

| ***Content of Formulation*** | ***Amount (%)*** |
|---|---|
| Ceftibuten dihydrate | 15% to 75% |
| Sodium bicarbonate | 15% to 50% |
| Citric acid | 10% to 45% |
| Pullulan | 0.1% to 15% |
| Sucralose | 0.05% to 10% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 5

| ***Content of Formulation*** | ***Amount (%)*** |
|---|---|
| Ceftibuten dihydrate | 22% to 50% |
| Sodium bicarbonate | 15% to 50% |
| Citric acid | 10% to 45% |
| Pullulan | 0.5% to 10% |
| Sucralose | 0.1% to 2% |
| PEG 8000 | 0.5% to 20% |
| Orange flavor | 0.05% to 15% |
| Lemon flavor | 0.05% to 15% |
| Sodium stearyl fumarate | 0.001% to 15% |
| Xanthan gum | 0.05% to 15% |
| Sodium lauryl sulfate | 0.005% to 5% |

### Example 6

| ***Content of Formulation*** | ***Amount (%)*** |
|---|---|
| Ceftibuten dihydrate | 10% to 90% |
| Sodium bicarbonate | 15% to 50% |
| Citric acid | 10% to 45% |
| Pullulan | 0.1% to 15% |
| Sucralose | 0.01 % to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 7

| ***Content of Formulation*** | ***Amount (%)*** |
|---|---|
| Ceftibuten dihydrate | 10% to 90% |
| Sodium bicarbonate | 15% to 50% |
| Citric acid | 10% to 45% |
| Pullulan | 0.5% to 10% |
| Sucralose | 0.01 % to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 8

| ***Content of Formulation*** | ***Amount (%)*** |
|---|---|
| Ceftibuten dihydrate | 22% to 50% |
| Sodium bicarbonate | 15% to 50% |
| Citric acid | 10% to 45% |
| Pullulan | 0.1% to 15% |
| Sucralose | 0.01% to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 9

| ***Content of Formulation*** | ***Amount (%)*** |
|---|---|
| Ceftibuten dihydrate | 22% to 50% |
| Sodium bicarbonate | 15% to 50% |
| Citric acid | 10% to 45% |
| Pullulan | 0.5% to 10% |
| Sucralose | 0.01% to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 10

Defined amounts of ceftibuten trihydrate with a mean particle size (d_{0.5}) of 4 µm to 25 µm as measured using a Malvern Mastersizer 2000 Particle Size Analyzer, sodium bicarbonate, citric acid (preferably nonaqueous), pullulan, sucralose, PEG 8000, orange flavor, lemon flavor, xanthan gum and sodium lauryl sulfate are weighed, these ingredients are introduced into a mixer and stirred therein for 20 minutes (first mixture). A previously weighed amount of sodium stearyl fumarate is added by sieving through a 630 µm sieve into the first mixture and the resulting mixture is taken into a mixer and stirred therein for 5 minutes (second mixture), then the resulting uniform mixture obtained in the final stirring step is compressed such that each effervescent tablet has a hardness of between 50 N and 250 N, and the compressed tablets are filled into blisters and packaged accordingly.

The production method described above can be adapted to all examples given below.

| ***Content of Formulation*** | ***Amount (%)*** |
|---|---|
| Ceftibuten trihydrate | 10% to 90% |
| Sodium bicarbonate | 15% to 50% |
| Citric acid | 10% to 45% |
| Pullulan | 0.05% to 25% |
| Sucralose | 0.05% to 10% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 11

| ***Content of Formulation*** | ***% Range*** |
|---|---|
| Ceftibuten trihydrate | 15% to 75% |
| Sodium bicarbonate | 18% to 45% |
| Citric acid | 12% to 40% |
| Pullulan | 0.1% to 15% |
| Sucralose | 0.1% to 5% |
| PEG 8000 | 1 % to 18% |
| Orange flavor | 0.1% to 10% |
| Lemon flavor | 0.1% to 10% |
| Sodium stearyl fumarate | 0.005% to 4% |
| Xanthan gum | 0.1% to 10% |
| Sodium lauryl sulfate | 0.01% to 4% |

### Example 12

| ***Content of Formulation*** | ***% Range*** |
|---|---|
| Ceftibuten trihydrate | 22% to 50% |
| Sodium bicarbonate | 20% to 40% |
| Citric acid | 15% to 35% |
| Pullulan | 0.5% to 10% |
| Sucralose | 0.1% to 2% |
| PEG 8000 | 2% to 15% |
| Orange flavor | 0.1% to 5% |
| Lemon flavor | 0.1% to 5% |
| Sodium stearyl fumarate | 0.005% to 3% |
| Xanthan gum | 0.1% to 5% |
| Sodium lauryl sulfate | 0.01% to 3% |

### Example 13

| ***Content of Formulation*** | ***Amount (%)*** |
|---|---|
| Ceftibuten trihydrate | 15% to 75% |
| Sodium bicarbonate | 15% to 50% |
| Citric acid | 10% to 45% |
| Pullulan | 0.1% to 15% |
| Sucralose | 0.1% to 5% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 14

| ***Content of Formulation*** | ***Amount (%)*** |
|---|---|
| Ceftibuten trihydrate | 22% to 50% |
| Sodium bicarbonate | 15% to 50% |
| Citric acid | 10% to 45% |
| Pullulan | 0.5% to 10% |
| Sucralose | 0.1% to 2% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 15

| ***Content of Formulation*** | ***Amount (%)*** |
|---|---|
| Ceftibuten trihydrate | 10% to 90% |
| Sodium bicarbonate | 15% to 50% |
| Citric acid | 10% to 45% |
| Pullulan | 0.1% to 15% |
| Sucralose | 0.05% to 10% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 16

| ***Content of Formulation*** | ***Amount (%)*** |
|---|---|
| Ceftibuten trihydrate | 10% to 90% |
| Sodium bicarbonate | 15% to 50% |
| Citric acid | 10% to 45% |
| Pullulan | 0.5% to 10% |
| Sucralose | 0.1% to 5% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 17

| ***Content of Formulation*** | ***Amount (%)*** |
|---|---|
| Ceftibuten trihydrate | 22% to 50% |
| Sodium bicarbonate | 15% to 50% |
| Citric acid | 10% to 45% |
| Pullulan | 0.1% to 15% |
| Sucralose | 0.01 % to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

### Example 18

| ***Content of Formulation*** | ***Amount (%)*** |
|---|---|
| Ceftibuten trihydrate | 22% to 50% |
| Sodium bicarbonate | 15% to 50% |
| Citric acid | 10% to 45% |
| Pullulan | 0.5% to 10% |
| Sucralose | 0.01% to 15% |
| PEG 8000 | 0.5% to 20% |
| Orange flavor | 0.05% to 15% |
| Lemon flavor | 0.05% to 15% |
| Sodium stearyl fumarate | 0.001% to 5% |
| Xanthan gum | 0.05% to 15% |
| Sodium lauryl sulfate | 0.005% to 5% |

Finally, all exemplary formulations given above for effervescent tablets can also be used for producing sachets formulations and effervescent granules according to the present invention.

## Claims

1. An effervescent formulation of ceftibuten or a pharmaceutically acceptable form thereof comprising pullulan.

2. The effervescent formulation according to Claim 1, wherein the amount of pullulan is 0.05% to 25% of the total weight of the formulation.

3. The effervescent formulation according to any of the preceding claims, wherein the ratio of ceftibuten to pullulan is between 1:10 and 100:1 by weight.

4. The effervescent formulation according to any of the preceding claims, wherein the amount of ceftibuten is 10% to 90% of the total weight of the formulation.

5. The effervescent formulation according to Claim 1, wherein a pharmaceutically acceptable form of ceftibuten is an amorphous form, a crystalline form, a free base form, a hydrate form, or a mixture of these forms, wherein ceftibuten is preferably in a dihydrate form, a trihydrate form, or a mixture of these forms.

6. The effervescent formulation according to any of the preceding claims, wherein the median particle size of ceftibuten is above 2 µm.

7. The effervescent formulation according to Claim 6, wherein the median particle size of ceftibuten is between 3 µm and 50 µm.

8. The effervescent formulation according to any of the preceding claims, further comprising at least one pharmaceutically acceptable excipient selected from a group comprising binders, sweeteners, glidants, effervescent pairs, surface active agents, disintegrants, flavoring agents, and viscosity agents.

9. The effervescent formulation according to Claim 8, wherein said sweeteners comprise sucralose, ammonium glycyrrhizinate, acesulfame-K, aspartame, saccharine or sodium salts or calcium salts of saccharine, sodium cyclamate, sucrose, fructose, glucose, sorbitol, or the mixtures thereof, wherein the sweetener is preferably sucralose.

10. The effervescent formulation according to Claim 9, wherein the amount of sucralose is 0.01% to 15% of the total weight of the formulation.

11. The effervescent formulation according to any of the preceding claims is in the form of an effervescent tablet, a sachet, or an effervescent granule.

12. The effervescent formulation according to any of the preceding claims, comprising the following ingredients:
a. 10 to 90% by weight of ceftibuten,
b. 15 to 50% by weight of sodium bicarbonate,
c. 10 to 45% by weight of citric acid,
d. 0.05 to 25% by weight of pullulan,
e. 0.01 to 15% by weight of sucralose,
f. 0.5 to 20% by weight of PEG 8000,
g. 0.05 to 15% by weight of orange flavor,
h. 0.05 to 15% by weight of lemon flavor,
i. 0.001 to 5% by weight of sodium stearyl fumarate,
j. 0.05 to 15% by weight of xanthan gum,
k. 0.005 to 5% by weight of sodium lauryl sulfate.

13. A method for preparing the effervescent formulation according to any of the preceding claims, comprising the steps of
a. weighing defined amounts of ceftibuten, sodium bicarbonate, citric acid, pullulan, sucralose, PEG 8000, orange flavor, lemon flavor, xanthan gum, and sodium lauryl sulfate, introducing these ingredients into a mixer and stirring therein (first mixture),
b. adding a previously weighed amount of sodium stearyl fumarate by sieving through a sieve into the first mixture and taking the resulting mixture into a mixer and stirring therein (second mixture),
c. subjecting the resulting uniform mixture obtained in the final stirring step to a filling and packaging process.
